# EUROPEAN PATENT APPLICATION

(11) **EP 0 960 600 A1**
(43) Date of publication of application: **01.12.1999**
(21) Application number: 99201416.7
(22) Date of filing: 06.05.1999
(51) Int. Cl.: A61B 17/06

(54) **Juncture between a surgical suture made of silicone elastomer and a needle**

(30) Priority: 29.05.1998 FI 981203
(71) Applicant: Nordström, Rolf E.A., 00120 Helsinki (FI)
(72) Inventor: Nordström, Rolf E.A., 00120 Helsinki (FI)
(74) Representative: LEITZINGER OY

(57) **Abstract**

The invention relates to a juncture between a surgical suture (2) made of silicone elastomer and a needle (1). The needle (1) has its end fitted with a long and thin gripping extension (7), which is provided with gripping barbs or through-going voids or with an outer surface that is coarse, rough or fibrous. The silicone suture is polymerized around the gripping extension (7) so as to establish a shape-clamped contact with the gripping extension (7). In the polymerization process, the juncture-zone silicone can be made less flexible than the actual silicone suture (2).

## Description

A suture made of silicone elastomer, which has been described in the Applicant's European patent application EP-792622, has proven particularly useful in stitching up even extensive skin areas or areas surgically removed because of skin deformations, for example. The usefulness of a suture made of silicone elastomer is based on the fact that it has a high elastic elongation, even though the elongating force remains relatively low and substantially stable. The suture is also suitable for closing up conventional surgical wounds, as it can be used for precluding the expansion of a scar and the migration of a suture through tissues.

Securing a silicone suture to a needle has turned out to be a nuisance. Due to a thickness and high elasticity of the suture, the conventional fastening methods are not capable of securing the suture in a sufficiently reliable manner.

An object of the invention is to provide a juncture, whereby a surgical suture made of silicone elastomer can be secured reliably to a needle.

This object is achieved by means of a juncture of the invention, which bears the characterizing features set forth in claim 1 or claim 2. The non-independent claims disclose preferred embodiments of the invention.

The invention will now be described by way of exemplary embodiments with reference made to the accompanying drawings, in which
- figs. 1 and 1A: depict a juncture according to a first embodiment of the invention;
- figs. 2 and 2A: depict a juncture according to a second embodiment of the invention; and
- figs. 3 and 3A: depict a juncture according to a third embodiment of the invention.

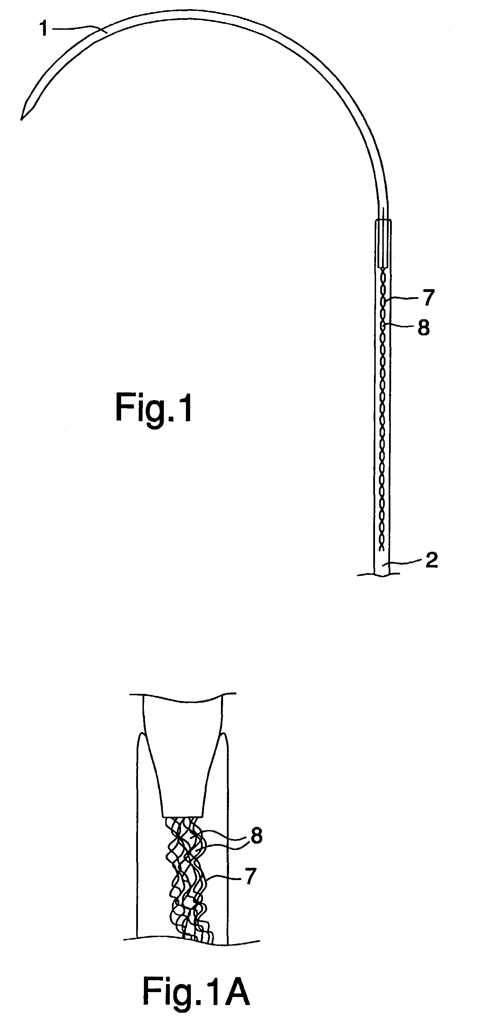

In the case of fig. 1, to the end of a needle 1 is fastened the length of 2-5 cm of a braided suture or filament 7. The question may be about a conventional, commercially available, surgical suture, the attachment of which to the end of the needle 1 is not a problem, it being routinely performed very successfully. The end of the filament 7 adheres inside a hollow base portion 9 of the needle.

Thus, the filament 7 constitutes a gripping extension for a silicone suture 2. What is essential is that the gripping extension 7 has a coarse, rough or fibrous surface, around which the end of the silicone suture 2 is polymerized in such a manner that the material of the silicone suture 2 conforms to a grip with scores or fibers present in the surface of the gripping extension 7. It is particularly beneficial to employ a sparsely twisted or braided filament, the silicone assuming the form of strands in the interstices of its braids. The filament or braid constituting the gripping extension 7 may consist of thin textile or artificial fibers.

If the shaped locking between the gripping extension 7 and the silicone suture 2 is not sufficiently effective, the attachment can be further enhanced by polymerizing the silicone suture around the gripping extension 7 in such a manner that the result of a polymerization process is that the silicone within the juncture zone becomes more inflexible (less flexible) than the actual silicone suture 2 outside the juncture zone. This change of inflexibility may take place gradually at the trailing end of the juncture zone, whereby an elongation of the suture 2 does not apply a disrupting stress to the juncture zone.

When the braid 7 serving as a gripping extension is designed to be sufficiently sparse, it develops through-going voids 8, to which the silicone strands effectively adhere.

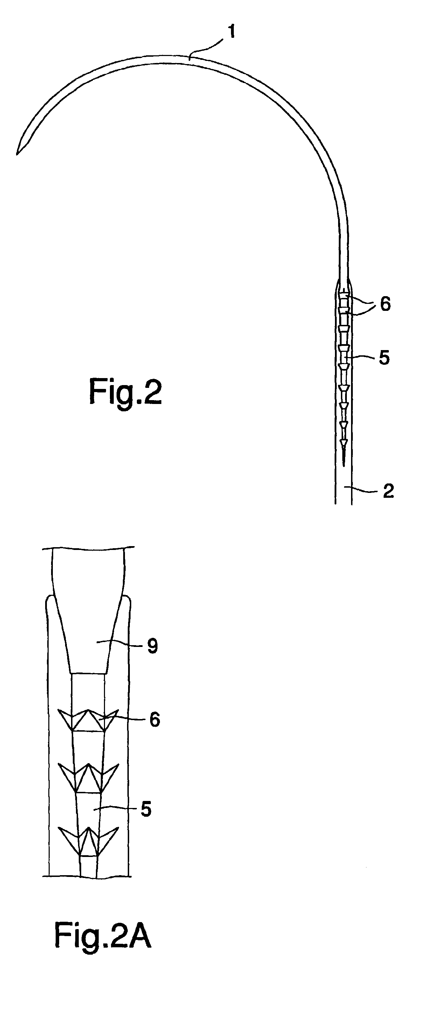

Fig. 2 depicts an alternative embodiment for the invention, wherein a gripping extension 5 made of a flexible or resilient metal is provided with a number of successive gripping barbs 6. In this case as well, the adherence can be enhanced by increasing the inflexibility of the silicone within the juncture zone, as the suture 2 still in a polymerization process is pressed around the gripping extension 5, 6.

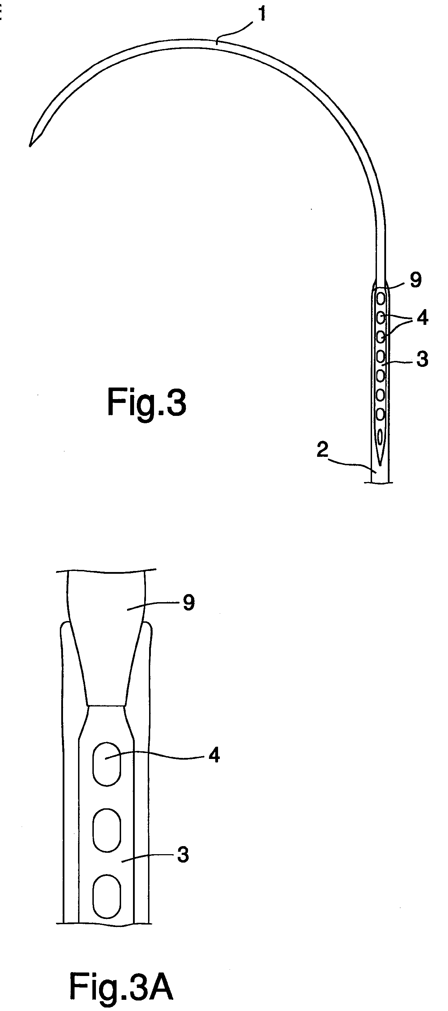

Fig. 3 depicts a ladder-shaped gripping extension 3 made of a metal or an artificial material, provided with a number of successive voids 4, through which the silicone material of the suture 2 builds gripping bridges. In this case as well, the adherence or gripping can be enhanced by making the silicone in the juncture zone more inflexible than elsewhere in the suture 2.

In the cases of figs. 2 and 3, the gripping extension can also be designed integrally with a needle 1. However, the materials of the needle and the gripping extension can be required to have different flexibility characteristics, those elements being manufactured separately from materials selected in accordance with required characteristics. In this case, it is also possible to use conventional standard-produced needles 1.

The manufacture of silicone sutures with a variety of stiffnesses is prior known technology, a further description of which is not necessary in this context. The additives for stiffening the juncture zone can be added in the juncture-zone silicone material after the actual silicone suture has been manufactured, but the polymerization process has not yet advanced substantially, whereby the silicone material is still soft and can be pressed around the gripping extension together with the added hardener.

## Claims

1. A juncture between a surgical suture (2) made of silicone elastomer and a needle (1), **characterized** in that the needle (1) has its end fitted with a long and thin gripping extension (3, 5, 7), which is provided with a number of successive gripping barbs (6) or through-going voids (4, 8) and around which is polymerized the silicone suture (2) in such a way that the suture material has shaped itself to establish a grip with the barbs (6) or through the voids (4, 8).

2. A juncture between a surgical suture (2) made of silicone elastomer and a needle (1), **characterized** in that the needle (1) has its end fitted with a long and thin gripping extension, which has a coarse, rough or fibrous surface and around which is polymerized the silicone suture (2) in such a way that the result of a polymerization process is such that the juncture-zone silicone becomes less flexible than the silicone suture (2).

3. A juncture as set forth in claim 1 or 2, **characterized** in that the gripping extension (7) is constituted by thin textile or artificial fibers.

4. A juncture as set forth in claim 3, **characterized** in that the gripping extension (7) comprises a sparsely twined filament, the silicone having shaped itself as strands in the interstices of the braids or fibers of such a filament.

5. A juncture as set forth in any of claims 1-4, **characterized** in that the gripping extension (3, 5, 7) has its end secured inside a hollow base portion (9) of the needle (1).

6. A juncture as set forth in any of claims 1-5, **characterized** in that the juncture zone has a length of 2-5 cm.

7. A juncture as set forth in claim 1, **characterized** in that the gripping extension (3) has a ladder-like appearance.

8. A juncture as set forth in claim 1, **characterized** in that the gripping extension is made of a flexible or resilient metal.

9. A juncture as set forth in claim 1, **characterized** in that in the polymerization process of the silicone suture (2), the juncture-zone silicone is made less flexible than the actual silicone suture (2).
